# EUROPÄISCHE PATENTSCHRIFT

(11) **EP 3 106 841 B1**
(45) Veröffentlichungstag und Bekanntmachung des Hinweises auf die Patenterteilung: **09.08.2017**
(21) Anmeldenummer: 15172631.2
(22) Anmeldetag: 18.06.2015
(51) Int. Cl.: G01D 11/24, G01N 27/22, G01N 33/00

(54) **UMKAPSELTER GASSENSOR**
ENCAPSULATED GAS SENSOR
CAPTEUR DE GAZ ENCAPSULÉ

(43) Veröffentlichungstag der Anmeldung: 21.12.2016
(73) Patentinhaber: E+E Elektronik Ges.m.b.H., 4209 Engerwitzdorf (AT)
(72) Erfinder: Haider, Albin, 4211 Alberndorf (AT)
(74) Vertreter: Hofmann, Ernst

(56) Entgegenhaltungen:
- EP-A1- 2 224 218
- DE-U1-202014 103 947
- US-A1- 2012 000 284
- US-A1- 2012 234 078

## Beschreibung

### GEBIET DER TECHNIK

Die vorliegende Erfindung betrifft eine Sensor-Vorrichtung.

### STAND DER TECHNIK

Aus der DE 20 2014 103 947 U1 ist eine Sensor-Vorrichtung bekannt. Diese umfasst einen integrierten Signalverarbeitungsbaustein, einen mit dem Signalverarbeitungsbaustein verbundenen gassensitiven Sensor sowie eine Umkapselung, die den Signalverarbeitungsbaustein mindestens teilweise umschließt. Über eine Kavität in der Umkapselung wird ein Zugang zum Sensor bereitgestellt.

Der Sensor kann beispielsweise als Feuchtesensor ausgebildet sein, so dass mit Hilfe einer derartigen Sensor-Vorrichtung Feuchtemessungen in unterschiedlichsten Umgebungen durchführbar sind.

Der Sensor ist in der Vorrichtung aus der genannten Druckschrift dabei mittig in der Umkapselung platziert; die Kavität erstreckt sich von der Umkapselungs-Oberseite trichterförmig verengend bis zum Sensor. Dies hat zur Folge, dass sich in der trichterförmigen Kavität und damit im Bereich des Sensors unter bestimmten Betriebsbedingungen Flüssigkeiten wie Kondenswasser oder sonstige Verunreinigungen, z.B. Chemikalien, ansammeln können. Während des üblicherweise nur sehr langsamen Abtrocknungsprozesses "sieht" der Sensor somit immer die Flüssigkeitsansammlung in der Kavität und liefert im Fall der Ausbildung als Feuchtesensor verfälschte Signale, die eine relative Feuchte von 100% suggerieren, was aber nicht der tatsächlichen Umgebungsfeuchte entspricht. Auch nach dem Abtrocknungsprozess können im Bereich der Kavität Rückstände der Verunreinigungen, beispielsweise Salze etc. verbleiben, die evtl. zukünftige Messungen verfälschen.

Aus der EP 2224218 A1 ist eine weitere Sensorvorrichtung bekannt, die einen integrierten Signalverarbeitungsbaustein, einen mit dem Signalverarbeitungsbaustein verbundenen gassensitiven Sensor sowie eine zylinderförmige Umkapselung umfasst, die den Signalverarbeitungsbaustein mindestens teilweise umschließt. An einem Ende der zylinderförmigen Umkapselung ist der Sensor für das umgebende Messgas zugänglich, wobei an diesem Ende eine Schutzkappe mit einem Filter vorgesehen ist, über die der Sensor vor einer eventuellen Beschädigung geschützt wird.

Die US 2012/0000284 A1 offenbart einen Feuchtigkeitssensor mit einem in eine Umkapselung integrierten Signalverarbeitungsbaustein. Die Umkapselung weist einen stufenförmigen Absatz auf, wobei in der unteren Stufenebene eine Öffnung für den darunter angeordneten Sensor vorgesehen ist.

### ZUSAMMENFASSUNG DER ERFINDUNG

Aufgabe der vorliegenden Erfindung ist es, eine Sensor-Vorrichtung anzugeben, die auch im Fall der Anlagerung von Flüssigkeiten und/oder Verunreinigungen im Bereich der Kavität einen störungsfreien Messbetrieb gewährleistet.

Diese Aufgabe wird durch eine Sensor-Vorrichtung mit den Merkmalen des Anspruchs 1 gelöst.

Vorteilhafte Ausführungsformen der erfindungsgemäßen Sensor-Vorrichtung ergeben sich aus den Maßnahmen, die in den von Anspruch 1 abhängigen Ansprüchen aufgeführt sind.

Die erfindungsgemäße Sensor-Vorrichtung umfasst einen integrierten Signalverarbeitungsbaustein, einen mit dem Signalverarbeitungsbaustein verbundenen gassensitiven Sensor sowie eine Umkapselung, die den Signalverarbeitungsbaustein mindestens teilweise umschließt, wobei eine.

Kavität in der Umkapselung einen Zugang zum Sensor bereitstellt. Die Umkapselung ist derart ausgebildet, dass sich die Kavität vom Sensor bis zu mindestens zwei begrenzenden Seitenflächen der Umkapselung erstreckt. Vorzugsweise erstreckt sich die Kavität vom Sensor bis zu zwei nicht-parallelen Seitenflächen der Umkapselung. Erfingdungsgemäß erstreckt sich die Kavität in der Umkapselung von einer ersten Seitenfläche der Umkapselung bis zu einer Kavitäts-Grundfläche, in der der Sensor angeordnet ist, wobei die Seitenwände der Kavität nicht senkrecht zur Kavitäts-Grundfläche angeordnet sind.
Es ist möglich, dass die Kavitäts-Grundfläche parabelförmig ausgebildet ist, wobei der Sensor im Bereich des Parabel-Scheitels angeordnet ist und sich die Parabel-Äste in Richtung einer zweiten Seitenfläche der Umkapselung erstrecken.
Desweiteren kann vorgesehen sein, dass
- die Umkapselung quaderförmig ausgebildet ist und
- die Kavität sich in Längsrichtung trichterförmig verengend von einer ersten Quader-Seitenfläche bis zu einer Kavitätsgrundfläche erstreckt und
- die Kavität sich in Querrichtung bis zu einer zweiten Quader-Seitenfläche erstreckt, wobei die erste und zweite Quader-Seitenfläche nicht-parallel zueinander orientiert sind.
Ferner ist möglich, dass der Sensor nicht-mittig in der Umkapselung angeordnet ist.
Vorzugsweise ist die Umkapselung aus Epoxidharz ausgebildet.
In einer möglichen Ausführungsform ist der Signalverarbeitungsbaustein mit einer elektrisch leitfähigen Trägerstruktur verbunden, wobei die Umkapselung auch die Trägerstruktur mit Ausnahme mehrerer elektrisch leitfähiger Kontaktflächen umgibt.
Alternativ hierzu kann auch vorgesehen sein, dass der Signalverarbeitungsbaustein lediglich auf einer Seite mit der Umkapselung versehen ist und auf einer weiteren Seite mehrere elektrisch leitfähige Kontaktflächen aufweist. Erfindungsgemäß ist vorgesehen, dass der Sensor als Feuchtesensor ausgebildet ist und der Signalverarbeitungsbaustein dazu eingerichtet und ausgebildet ist, die vom Sensor erzeugten Signale in weiterverarbeitbare Feuchte-Messwerte umzuwandeln. Der Feuchtesensor weist eine Grundelektrode auf, auf der eine feuchteempfindliche Messschicht angeordnet ist, auf der sich eine Deckelektrode befindet, wobei der Feuchtesensor auf einer auf dem Signalverarbeitungsbaustein angeordneten Isolationsschicht platziert ist. Erfindungsgemäß ist vorgesehene, dass der Feuchtesensor eine feuchtedurchlässige Schutzschicht aufweist, die hydrophob ausgebildet ist.

Es ist ferner möglich, dass die Schutzschicht ein Gefälle in Richtung einer begrenzenden Seitenfläche der Kavität besitzt.
Als besonders vorteilhaft an der erfindungsgemäßen Sensor-Vorrichtung erweist sich, dass keine Rückstände durch das Abtrocknen von Flüssigkeitsansammlungen in der Kavität mehr entstehen, da die Flüssigkeit wegen der seitlichen Öffnung aus der Kavität rasch abfließen kann. Dadurch resultiert ferner auch eine schnelle Reaktionszeit der Sensor-Vorrichtung in Bezug auf die jeweilige Messgröße, z.B. relative Feuchte, da nach einer eventuellen Kondensation die sich in der Kavität angesammelte Flüssigkeit nicht erst langsam abtrocknen muss.

Als weiterer Vorteil der erfindungsgemäßen Maßnahmen ergibt sich die Möglichkeit, die Sensor-Vorrichtung in einer sehr flachen Bauweise auszuführen, da der Sensor nunmehr auch durch den seitlichen Zugang vom jeweiligen Gas beaufschlagt werden kann. Dadurch ergeben sich vielfältige weitere Einsatzmöglichkeiten für die erfindungsgemäße Sensor-Vorrichtung, beispielsweise in flachen elektronischen Mobilgeräten, an Windschutzscheiben in Kraftfahrzeugen usw..

Weitere Einzelheiten und Vorteile der vorliegenden Erfindung seien anhand der nachfolgenden Beschreibung von Ausführungsbeispielen der erfindungsgemäßen Sensor-Vorrichtung in Verbindung mit den Figuren erläutert.

### KURZE BESCHREIBUNG DER ZEICHNUNGEN

Es zeigt hierbei
- Figur 1a, 1b: jeweils eine perspektivische Ansicht eines Ausführungsbeispiels der erfindungsgemäßen Sensor-Vorrichtung;
- Figur 2a: eine Draufsicht auf das Ausführungsbeispiel der erfindungsgemäßen Sensor-Vorrichtung aus den Figuren 1a, 1b;
- Figur 2b: eine Seitenansicht des Ausführungsbeispiels der erfindungsgemäßen Sensor-Vorrichtung aus den Figuren 1a, 1b;
- Figur 3a: eine Schnittansicht des Ausführungsbeispiels der erfindungsgemäßen Sensor-Vorrichtung aus den Figuren 1a, 1b;
- Figur 3b: eine vergrößerte Darstellung eines Details aus Figur 3a;
- Figur 3c: eine vergrößerte Darstellung zum Aufbau des Sensors aus den Figuren 3a, 3b;
- Figur 4: eine schematisierte Darstellung zur Erläuterung der erfindungsgemäßen Sensor-Vorrichtung;
- Figur 5: eine Darstellung zur Erläuterung eines möglichen Applikationsbeispiels für die erfindungsgemäße Sensor-Vorrichtung;
- Figur 6a, 6b: jeweils eine perspektivische Ansicht eines weiteren Ausführungsbeispiels der erfindungsgemäßen Sensor-Vorrichtung.

### BESCHREIBUNG DER AUSFÜHRUNGSFORMEN

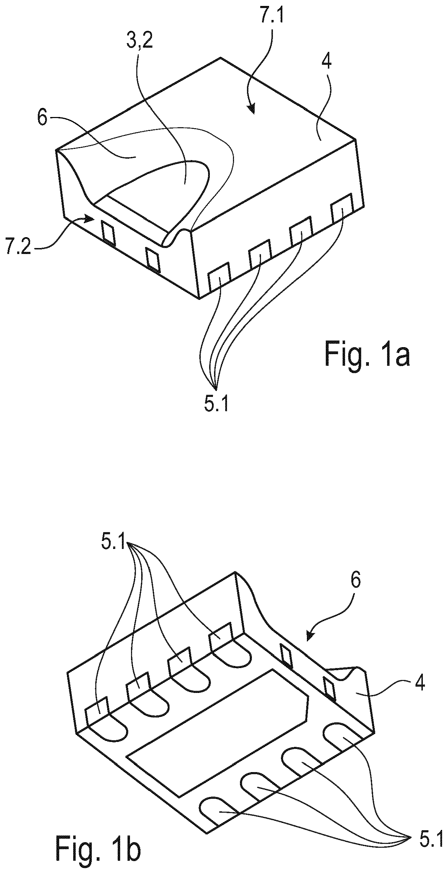

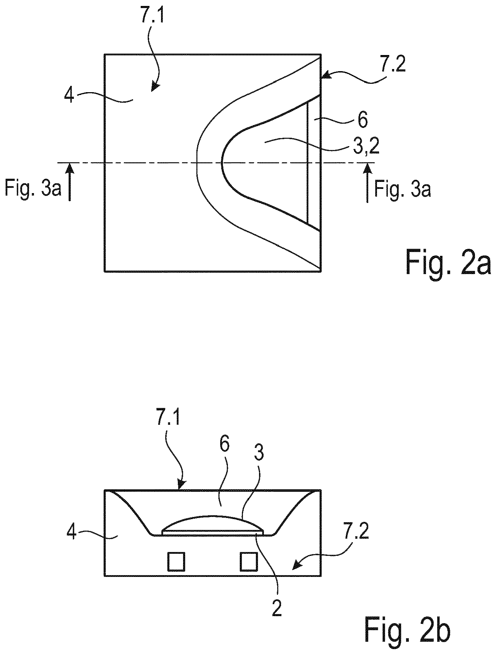

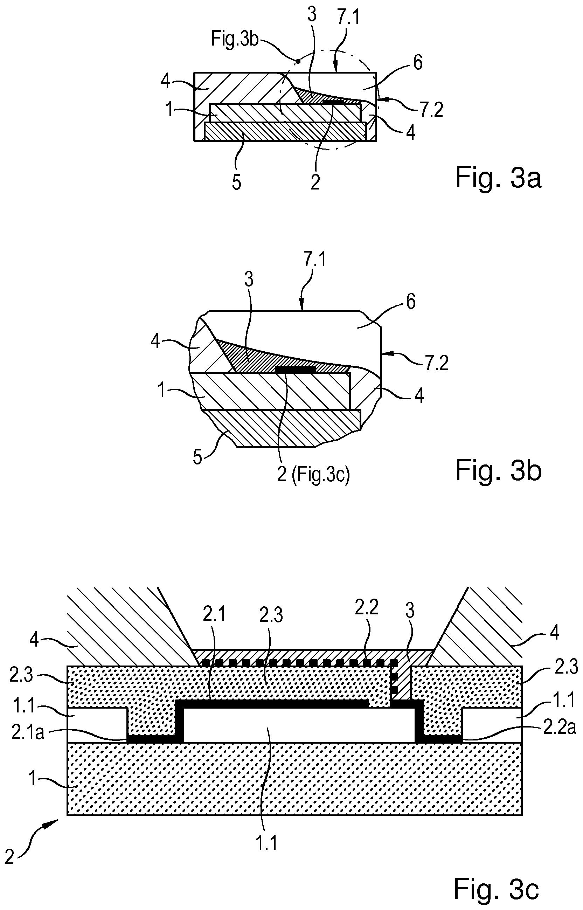

Ein erstes Ausführungsbeispiel der erfindungsgemäßen Sensor-Vorrichtung ist in den Figuren 1a, 1b jeweils in einer perspektivischen Ansicht gezeigt; die Figuren 2a, 2b zeigen eine Drauf- bzw. Seitenansicht dieses Ausführungsbeispiels, die Figuren 3a eine Schnittansicht entlang der in Figur 2a dargestellten Schnittlinie und die Figuren 3b, 3c weitere Details dieses Ausführungsbeispiels.

Das dargestellte Ausführungsbeispiel der erfindungsgemäßen Sensor-Vorrichtung umfasst einen integrierten Signalverarbeitungsbaustein 1 (ASIC), der auf einem elektrisch leitfähigen Trägerkörper 5 in Form eines sog. Lead-Frames angeordnet ist. Auf der Oberseite des Signalverarbeitungsbausteins 1 ist ein gassensitiver Sensor 2 platziert, der mit dem Signalverarbeitungsbaustein 1 elektrisch leitend verbunden ist. Oberhalb des Sensors 2 ist in dieser Ausführungsform ferner eine gasdurchlässige Schutzschicht 3 angeordnet, die hydrophobe bzw. wasserabweisende Eigenschaften aufweist.
Der Signalverarbeitungsbaustein 1 und der gassensitive Sensor 2 werden in der erfindungsgemäßen Sensor-Vorrichtung mindestens teilweise von einer Umkapselung 4 umschlossen. Die Formgebung der Umkapselung 4 erfolgt im Verlauf des Transfer-Molding-Herstellungsprozesses hierbei derart, dass eine Quaderform der Sensor-Vorrichtung resultiert; als Material für die Umkapselung 4 dient z.B. Epoxidharz. Im dargestellten Ausführungsbeispiel besitzt der Quader einen quadratischen Querschnitt mit identischer Länge und Breite. In der Umkapselung 4 ist desweiteren eine Kavität 6 vorgesehen, über die für das zu messende Gas ein Zugang zum Sensor 2 bereitgestellt wird.

Der Sensor 2 ist erfindungsgemäß als Feuchtesensor ausgebildet und liefert feuchteabhängige Signale, die vom Signalverarbeitungsbaustein 1 in weiterverarbeitbare Feuchtemesswerte umgewandelt werden. Die Feuchtemesswerte können dann von einer nachgeordneten, nicht dargestellten, Folgeelektronik in verschiedenster Art und Weise weiterverarbeitet werden; ein mögliches Applikationsbeispiel der erfindungsgemäßen Sensor-Vorrichtung wird im Verlauf der weiteren Beschreibung noch erläutert. Als vorteilhaft erweist sich dabei zur Signal-Weiterverarbeitung, wenn in den Signalverarbeitungsbaustein 1 ein oder mehrere Temperatursensoren integriert sind. Zur Verbindung mit einer Folgeelektronik weist die Trägerstruktur 5, auf der der Signalverarbeitungsbaustein 1 im vorliegenden Ausführungsbeispiel angeordnet ist, mehrere elektrisch leitfähige Kontaktflächen 5.1 bzw. Kontaktpads auf, die nicht von der Umkapselung 4 umgeben sind. Über diese Kontaktflächen kann der Signalverarbeitungsbaustein 1 z.B. mittels nicht dargestellter Bonddrähte elektrisch kontaktiert werden.
Im Hinblick auf die konkrete Ausgestaltung des als Feuchtesensor ausgebildeten Sensors 2 sei auf die später folgende Beschreibung der Figuren 3a - 3c verwiesen.

Wie insbesondere aus den Figuren 1a, 1b sowie 2a, 2b ersichtlich, ist die Umkapselung 4 erfindungsgemäß derart ausgebildet, dass sich die Kavität 6 ausgehend vom Sensor 2 bis zu mindestens zwei begrenzenden Seitenflächen 7.1, 7.2 der Umkapselung 4 erstreckt. Im vorliegenden Ausführungsbeispiel handelt es sich hierbei um die aus Sensorsicht obere, erste Seitenfläche 7.1 sowie um die seitlich nächstliegende, zweite Seitenfläche 7.2 der quaderförmigen Umkapselung 4, die nicht-parallel zur ersten Seitenfläche 7.1 orientiert ist. Die Kavität 6 verengt sich in Längsrichtung ausgehend von der ersten Seitenfläche 7.1 des Quaders trichterförmig hin zur Kavitäts-Grundfläche und besitzt in Richtung der zweiten Seitenfläche 7.2 der quaderförmigen Sensor-Vorrichtung eine seitliche Öffnung. Im dargestellten Ausführungsbeispiel sind die erste Seitenfläche 7.1 bzw. erste Quader-Seitenfläche und die zweite Seitenfläche 7.2 bzw. zweite Quader-Seitenfläche demzufolge nicht-parallel zueinander orientiert. Die Seitenwände der Kavität 6 sind ferner nicht senkrecht zur Kavitäts-Grundfläche angeordnet, sondern verlaufen ausgehend von der Kavitäts-Grundfläche schräg nach oben in Richtung der ersten Seitenfläche 7.1.

Die Kavitäts-Seitenwände bzw. die Kavitäts-Grundfläche sind im dargestellten Ausführungsbeispiel in etwa parabelförmig ausgebildet, wobei der Sensor 2 ungefähr im Bereich des Parabel-Scheitelpunkts angeordnet ist und die Parabel-Äste bzw. die Öffnung der Parabel sich in Richtung der zweiten Seitenfläche 7.2 erstrecken. In Querrichtung erstreckt sich die Kavität 6 somit bis zur zweiten Seitenfläche 7.2 des Quaders, der durch die Umkapselung 4 gebildet wird.

Über die vorstehend erläuterte, erfindungsgemäße Formgebung der Kavität 6, die an einer Seite nicht durch Seitenwände der Umkapselung 4 begrenzt ist, sondern hier eine Öffnung aufweist, wird somit sichergestellt, dass ggf. in der Kavität 6 befindliche Flüssigkeit rasch aus dieser abfließen kann. Eine Verfälschung von Sensor-Messwerten durch sich evtl. in der Kavität 6 ansammelnde Flüssigkeit wird zuverlässig verhindert.

In einem Ausführungsbeispiel der erfindungsgemäßen Sensor-Vorrichtung besitzt der Quader eine Länge bzw. eine Breite von 2,0mm sowie eine Höhe von 0,75mm. Der Abstand der Kavitäts-Grundfläche von der ersten Seitenfläche 7.1 bzw. der Quader-Oberseite beträgt 0,325mm. Die Parabel-Äste der Kavitäts-Grundfläche nehmen zueinander einen Winkel von ca. 50° ein. Der Anordnungswinkel der Seitenwände beträgt in etwa 60°, d.h. die Seitenwände sind ca. 30° gegenüber der Senkrechten verkippt.

Grundsätzlich ist bei der Dimensionierung der erfindungsgemäßen Sensor-Vorrichtung zu beachten, dass die Abmessungen sowie die verschiedenen Winkel und Schrägen der Umkapselung 4 so gewählt werden, dass diese möglichst gut zu fertigen ist und zudem eventuell erforderliche Bonddrähte ausreichend geschützt werden. Desweiteren sollte die Kavität 6 eine bestimmte Mindestgröße nicht unterschreiten, da ansonsten die Kapillarwirkung auf eine in der Kavität 6 befindliche Flüssigkeit den gewünschten Abfluss der Flüssigkeit aus der Kavität 6 verhindern könnte.

Anhand der Figuren 3a - 3c werden nachfolgend weitere Details des ersten Ausführungsbeispiels der erfindungsgemäßen Sensor-Vorrichtung erläutert.

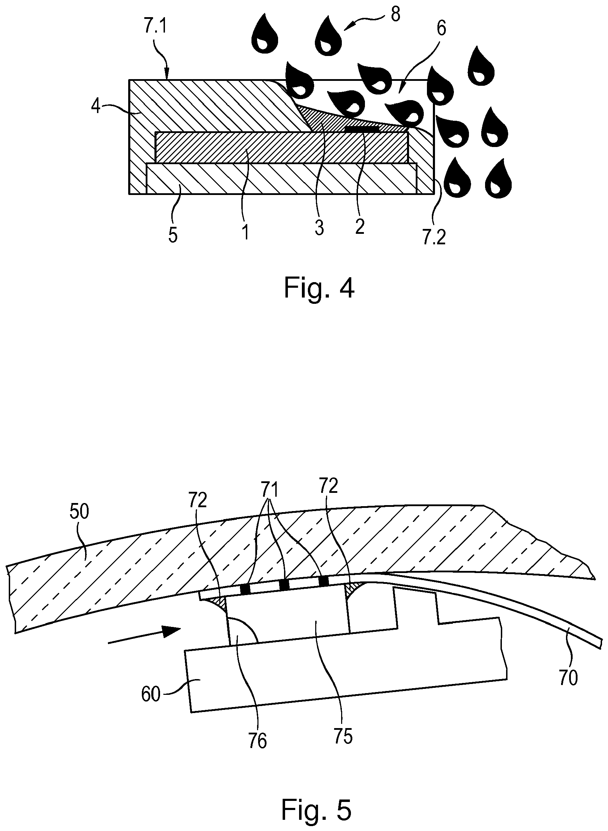

Aus der Schnittansicht der Figur 3a entlang der in Figur 2a eingezeichneten Schnittlinie sowie der Detailansicht in Figur 3 ist zum einen die gewählte Formgebung der Kavität 6 innerhalb der Umkapselung 4 mit den schräg verlaufenden Seitenwänden der Kavität 6 ersichtlich. Zum anderen ist daraus auch eine mögliche Ausbildung der Schutzschicht 3 oberhalb des hier nicht detaillierter dargestellten Sensors 2 erkennbar. So weist die Schutzschicht 3 ein Gefälle in Richtung der zweiten Seitenfläche 7.2 auf, d.h. die Höhe der Schutzschicht 3 über dem Sensor 2 nimmt ausgehend von der Seitenwand der Kavität 6 in Richtung der begrenzenden zweiten Seitenfläche 7.2 ab. Auf diese Art und Weise kann ein Abfließen von Flüssigkeit nochmals verbessert werden, die sich evtl. in der Kavität 6 ansammelt; vorteilhaft diesbezüglich wirkt sich auch die bereits oben erwähnte Wahl eines Schutzschichtmaterials mit hydrophoben Eigenschaften aus. Der entsprechende Effekt ist in Figur 4 schematisiert veranschaulicht, wo das resultierende Abfließen von Flüssigkeitstropfen 8 über die seitliche Öffnung der Kavität 6 an der zweiten Seitenfläche 7.2 in der erfindungsgemäßen Sensor-Vorrichtung gezeigt ist.

Eine Detail-Darstellung einer möglichen Ausführungsform des als kapazitiver Feuchtesensor ausgebildeten gassensitiven Sensors 2 ist in Figur 3c aus Sicht der Seitenfläche 7.2 gezeigt. Der Sensor 2, ausgebildet als Plattenkondensator, ist hierbei auf einer Isolationsschicht 1.1 auf dem Signalverarbeitungsbaustein 1 angeordnet und umfasst i.w. eine flächige Grundelektrode 2.1, die unmittelbar auf der Isolationsschicht 1.1 angeordnet ist, eine darüber angeordnete feuchteempfindliche Messschicht 2.3 sowie eine über der Messschicht 2.3 angeordnete flächige Deckelektrode 2.2, die mit Rissen versehen ist. Die Geometrie der Grund- und Deckelektrode 2.1, 2.2 ist im vorliegenden Ausführungsbeispiel jeweils kreisförmig gewählt, könnte alternativ aber auch eine andere Form aufweisen oder an die Form der Kavitäts-Grundfläche angepasst sein. Ebenfalls wäre alternativ hierzu eine Ausbildung der Grund- und/oder Deckelektrode als Interdigitalelektrode möglich.
Die Grundelektrode 2.1 besteht im vorliegenden Ausführungsbeispiel aus einem leitfähigen Material, z.B. Nickel; das Material für die Deckelektrode 2.2 ist ebenfalls elektrisch leitfähig und muss zudem gasdurchlässig sein, was vorliegend durch die Risse in der Deckelektrode 2.2 gewährleistet wird. Als Messschicht 2.3, deren Kapazität sich feuchteabhängig ändert, dient ein geeignetes Dielektrikum wie z.B. Polyimid.

Der Feuchtesensor 2 bzw. dessen Grundelektrode 2.1 und dessen Deckelektrode 2.2 sind elektrisch leitend mit dem Signalverarbeitungsbaustein 1 verbunden. Die auf der Isolationsschicht 1.1 des Signalverarbeitungsbausteins 1 angeordnete Grundelektrode 2.1 wird im dargestellten Beispiel über den links außen in Figur 3c gezeigten ersten Kontaktierungsbereich 2.1 a mit dem Signalverarbeitungsbaustein 1 verbunden, die Deckelektrode 2.2 ist über den rechts außen angeordneten zweiten Kontaktierungsbereich 2.2a mit dem Signalverarbeitungsbaustein 1 elektrisch leitend verbunden. Um die beiden Elektroden 2.1, 2.2 über entsprechende Zuleitungen mit diesen Kontaktierungsbereichen 2.1 a, 2.2a ist die ansonsten durchgängig auf dem Signalverarbeitungsbaustein 1 angeordnete Isolationsschicht 1.1 wie aus Figur 3c ersichtlich lokal unterbrochen. Aus der Bestimmung der sich feuchteabhängig ändernden Kapazität können über den Signalverarbeitungsbaustein 1 entsprechende Feuchte-Messwerte erzeugt werden.

Ein mögliches Applikationsbeispiel für die erfindungsgemäße Sensor-Vorrichtung ist in Figur 5 schematisiert veranschaulicht. Es handelt sich hierbei um den Einsatz an der Innenseite der Windschutzscheibe 50 eines Kraftfahrzeugs. Mit Hilfe der Sensor-Vorrichtung 75 wird hier die Feuchte und Temperatur an der Oberfläche der Windschutzscheibe 50 bestimmt, um einem eventuellen Beschlagen durch geeignete klimatechnische Maßnahmen entgegenzuwirken. Die Sensor-Vorrichtung 75 wird hierzu möglichst nah an der Windschutzschiebe 50 platziert, wozu die Sensor-Vorrichtung auf einer flexiblen Platine 70 angeordnet ist, die im Anordnungsbereich der Windschutzscheibe 50 auf dieser platziert wird. Die flexible Platine weist mehrere Durchkontaktierungen 71 auf, die mit Kupfer-Material verfüllt sind und eine gute thermische Anbindung der Sensor-Vorrichtung 75 an die Windschutzscheibe 50 gewährleisten; desweiteren wird über die flexible Platine 70 die Verbindung der Sensor-Vorrichtung 75 mit einer nicht dargestellten Folgeelektronik sichergestellt. Die Sensor-Vorrichtung 75 als auch die flexible Platine 70 werden im entsprechenden Anordnungsbereich eines Rückspiegelgehäuses 60 verklemmt bzw. fixiert, der wiederum über eine nicht dargestellte Klebefolie an der Windschutzscheibe 50 befestigt ist.

Wie aus Figur 5 ersichtlich, kann über die erfindungsgemäße Formgebung der Kavität 76 in der Sensor-Vorrichtung 75 bei einem sehr flach bauenden Aufbau der Zugang des zu messenden Gases zum Sensor gewährleistet werden. Über die seitliche Öffnung der Kavität 76 kann das Messgas den Sensor ungehindert erreichen. Als weitere Optimierungsmaßnahme wäre es dabei möglich, im Rückspiegelgehäuse 60 oberhalb der Kavität der Sensor-Vorrichtung eine Öffnung vorzusehen, durch die das zu messende Gas entweichen kann und sich nicht am Sensor der Sensor-Vorrichtung staut. Auf diese Art und Weise kann im Bedarfsfall eine schnellere Ansprechzeit des Sensors erreicht werden.

Neben einer derartigen Anwendung gibt es selbstverständlich vielfältigste weitere Applikationsmöglichkeiten für die erfindungsgemäße Sensor-Vorrichtung, insbesondere wenn es ähnlich wie im vorliegenden Beispiel auf eine möglichst flach bauende Bauform ankommt und ein seitlicher Einlass für das Messgas vorteilhaft ist. Hierbei kann es sich etwa um die Integration der Sensor-Vorrichtung in flache elektronische Mobilgeräte handeln wie beispielsweise Smartphones, die damit zur Gas-Detektion nutzbar wären.

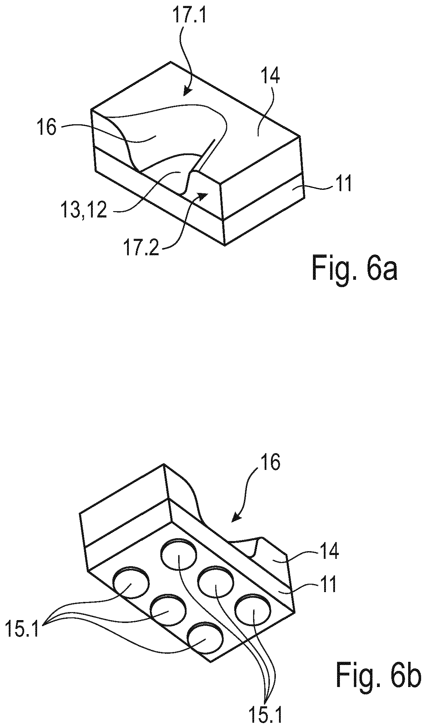

Ein zweites Ausführungsbeispiel der erfindungsgemäßen Sensor-Vorrichtung ist in den Figuren 6a, 6b in analogen Darstellungen zum ersten Ausführungsbeispiel gezeigt. Nachfolgend seien i.w. nur die maßgeblichen Unterschiede zum ersten Ausführungsbeispiel erläutert.

So ist nunmehr nicht vorgesehen den Signalverarbeitungsbaustein 11 auf einem Trägerkörper bzw. Lead-Frame anzuordnen und darüber elektrisch zu kontaktieren; vielmehr erfolgt die elektrische Kontaktierung unmittelbar über die auf der Unterseite des Signalverarbeitungsbausteins 11 angeordneten Kontaktflächen 15.1 in Form sog "Balls", über die der Signalverarbeitungsbaustein 11 direkt auf eine Platine gelötet werden kann.

Der Signalverarbeitungsbaustein 11 ist hier lediglich auf einer Seite mit der Umkapselung 14 versehen, nämlich auf der Oberseite, wo auch wiederum der gassensitive Sensor 12 inclusive Schutzschicht 13 angeordnet ist. Die Formgebung der Umkapselung 14 und insbesondere die geometrische Ausbildung der Kavität 16 erfolgt analog zum ersten Ausführungsbeispiel, wobei der Quader in diesem Beispiel keinen quadratischen Querschnitt besitzt, sondern rechteckförmig ausgebildet ist.

Neben den erläuterten Ausführungsbeispielen existieren im Rahmen der vorliegenden Erfindung selbstverständlich noch weitere Ausgestaltungsmöglichkeiten.

So gibt es etwa in Bezug auf die Geometrie der Kavität im Rahmen der vorliegenden Erfindung auch alternative Realisierungsmöglichkeiten. So kann die Kavitäts-Grundfläche etwa auch halbkreisförmig, quadratisch oder rechteckig ausgebildet werden. Selbstverständlich ist es auch möglich, den Anordnungswinkel der Seitenwände der Kavität anders zu wählen als im oben erläuterten Ausführungsbeispiel.

Desweiteren ist es möglich, die in der Kavität vorgesehene Schutzschicht oberhalb des Feuchtesensors wegzulassen und stattdessen vor dem Trennen bzw. Vereinzeln der im Nutzen gefertigten Sensor-Vorrichtung auf die gesamte Nutzen-Oberfläche eine hydrophobe Beschichtung mit einer Schichtdicke im Bereich von 100 nm z.B. über ein Spin-Coating-Verfahren aufzubringen. Diese Beschichtung würde dann die Oberseite einer vereinzelten erfindungsgemäßen Sensor-Vorrichtung vollständig bedecken, d.h. sowohl die Umkapselung als auch die Kavität. Nach dem Vereinzeln der Sensor-Vorrichtungen, z.B. über Sägen oder Laser-Schneiden, liegen an den Seitenflächen der Umkapselung vorgesehene Kontaktflächen für den Signalverarbeitungsbaustein frei und können etwa über Bonddrähte elektrisch kontaktiert werden.

Die vorstehend erwähnte Beschichtung könnte darüber hinaus auch auf der Oberseite eines Sensor-Vorrichtungsaufbaus aufgebracht werden, wie er im ersten Ausführungsbeispiel erläutert wurde und bei dem die Schutzschicht oberhalb des Sensors nicht weggelassen wird.

In weiteren alternativen Einbauvarianten kann vorgesehen werden, an der Kante der Sensorvorrichtung zwischen der ersten und zweiten Seitenfläche der Umkapselung eine Abschottungswand vorzusehen. Dadurch gelangt der Strom mit dem Messgas von einer Seitenwand durch die Kavität zur anderen Seitenwand, d.h. der gesamte Strom mit dem Messgas streift dann über den Sensor in der Kavität, was eine schnelle Ansprechzeit des Sensors ermöglicht.

## Patentansprüche

1. Sensor-Vorrichtung mit
- einem integrierten Signalverarbeitungsbaustein (1),
- einem mit dem Signalverarbeitungsbaustein (1) verbundenen gassensitiven Sensor (2; 12),
- einer Umkapselung (4; 14), die den Signalverarbeitungsbaustein (1) mindestens teilweise umschließt, wobei eine Kavität (6; 16) in der Umkapselung (4; 14) einen Zugang zum Sensor (2; 12) bereitstellt und die Umkapselung (4; 14) derart ausgebildet ist, dass sich die Kavität (6; 16) vom Sensor (2; 12) bis zu mindestens zwei begrenzenden Seitenflächen (7.1, 7.2) der Umkapselung (4; 14) erstreckt, wobei sich die Kavität (6; 16) in der Umkapselung (4; 14) von einer ersten Seitenfläche (7.1) der Umkapselung (4; 14) bis zu einer Kavitäts-Grundfläche erstreckt, in der der Sensor (2; 12) angeordnet ist, wobei die Seitenwände der Kavität (6; 16) nicht senkrecht zur Kavitäts-Grundfläche angeordnet sind und
- wobei der Sensor (2; 12) als Feuchtesensor ausgebildet ist und der Signalverarbeitungsbaustein (1; 11) dazu eingerichtet und ausgebildet ist, die vom Sensor (2; 12) erzeugten Signale in weiterverarbeitbare Feuchte-Messwerte umzuwandeln und **dadurch gekennzeichnet dass** der Feuchtesensor eine Grundelektrode (2.1) aufweist, auf dem eine feuchteempfindliche Messschicht (2.3) angeordnet ist, auf dem sich eine Deckelektrode (2.2) mit einer feuchtedurchlässigen hydrophoben Schutzschicht (3; 13) befindet und wobei der Feuchtesensor auf einer auf dem Signalverarbeitungsbaustein (1; 11) angeordneten Isolationsschicht (1.1) platziert ist.

2. Sensor-Vorrichtung nach Anspruch 1, wobei sich die Kavität (6; 16) vom Sensor (2; 12) bis zu zwei nicht-parallelen Seitenflächen (7.1, 7.2) der Umkapselung (4; 14) erstreckt.

3. Sensor-Vorrichtung nach Anspruch 1, wobei die Kavitäts-Grundfläche parabelförmig ausgebildet ist, wobei der Sensor (2; 12) im Bereich des Parabel-Scheitels angeordnet ist und sich die Parabel-Äste in Richtung einer zweiten Seitenfläche (7.2) der Umkapselung (4; 14) erstrecken.

4. Sensor-Vorrichtung nach Anspruch 1, wobei
- die Umkapselung (4; 14) quaderförmig ausgebildet ist und
- die Kavität (6; 16) sich in Längsrichtung trichterförmig verengend von einer ersten Quader-Seitenfläche (7.1) bis zu einer Kavitätsgrundfläche erstreckt und
- die Kavität (6; 16) sich in Querrichtung bis zu einer zweiten Quader-Seitenfläche (7.2) erstreckt, wobei die erste und zweite Quader-Seitenfläche (7.1, 7.2) nicht-parallel zueinander orientiert sind.

5. Sensor-Vorrichtung nach mindestens einem der vorhergehenden Ansprüche, wobei der Sensor (2; 12) nicht-mittig in der Umkapselung (4; 14) angeordnet ist.

6. Sensor-Vorrichtung nach mindestens einem der vorhergehenden Ansprüche, wobei die Umkapselung (4; 14) aus Epoxidharz ausgebildet ist.

7. Sensor-Vorrichtung nach mindestens einem der vorhergehenden Ansprüche, wobei der Signalverarbeitungsbaustein (1) mit einer elektrisch leitfähigen Trägerstruktur (5) verbunden ist und die Umkapselung (4) auch die Trägerstruktur (5) mit Ausnahme mehrerer elektrisch leitfähiger Kontaktflächen (5.1) umgibt.

8. Sensor-Vorrichtung nach mindestens einem der Ansprüche 1 - 6, wobei der Signalverarbeitungsbaustein (11) lediglich auf einer Seite mit der Umkapselung (14) versehen ist und auf einer weiteren Seite mehrere elektrisch leitfähige Kontaktflächen (15.1) aufweist.

9. Sensor-Vorrichtung nach Anspruch 1, wobei die Schutzschicht (3; 13) ein Gefälle in Richtung einer begrenzenden Seitenflächen (7.2) der Kavität (6; 16) besitzt.

## Claims

1. Sensor device comprising
- an integrated signal processing module (1),
- a gas sensitive sensor (2; 12) connected to the signal processing module (1),
- an encapsulation (4; 14) which at least partly encloses the signal processing module (1), a cavity (6; 16) in the encapsulation (4; 14) providing an access to the sensor (2; 12), and the encapsulation (4; 14) being formed in such a way that the cavity (6; 16) extends from the sensor (2; 12) as far as at least two limiting side surfaces (7.1, 7.2) of the encapsulation (4; 14), the cavity (6; 16) in the encapsulation (4; 14) extending from a first side surface (7.1) of the encapsulation (4; 14) as far as a cavity base surface, in which the sensor (2; 12) is arranged, the side walls of the cavity (6; 16) not being arranged at right angles to the cavity base surface, and
- the sensor (2; 12) being formed as a moisture sensor and the signal processing module (1; 11) being configured and formed to convert the signals generated by the sensor (2; 12) into moisture measured values that can be processed further, and
**characterized in that**
the moisture sensor has a base electrode (2.1) on which a moisture-sensitive measuring layer (2.3) is arranged, on which there is a top electrode (2.2) with a moisture-permeable hydrophobic protective layer (3; 13), and wherein the moisture sensor is placed on an insulating layer (1.1) arranged on the signal processing module (1; 11).

2. Sensor device according to Claim 1, wherein the cavity (6; 16) extends from the sensor (2; 12) as far as two non-parallel side surfaces (7.1, 7.2) of the encapsulation (4; 14).

3. Sensor device according to Claim 1, wherein the cavity base surface is formed in the shape of a parabola, wherein the sensor (2; 12) is arranged in the area of the vertex of the parabola and the branches of the parabola extend in the direction of a second side surface (7.2) of the encapsulation (4; 14).

4. Sensor device according to Claim 1, wherein
- the encapsulation (4; 14) is formed in the shape of a cube and
- the cavity (6; 16) narrows in the shape of a funnel in the longitudinal direction from a first cube side surface (7.1) as far as a cavity base surface, and
- the cavity (6; 16) extends in the transverse direction as far as a second cube side surface (7.2), wherein the first and second cube side surfaces (7.1, 7.2) are oriented non-parallel to each other.

5. Sensor device according to at least one of the preceding claims, wherein the sensor (2; 12) is arranged non-centrally in the encapsulation (4; 14).

6. Sensor device according to at least one of the preceding claims, wherein the encapsulation (4; 14) is formed from epoxy resin.

7. Sensor device according to at least one of the preceding claims, wherein the signal processing module (1) is connected to an electrically conductive carrier structure (5), and the encapsulation (4) also surrounds the carrier structure (5) with the exception of a plurality of electrically conductive contact surfaces (5.1).

8. Sensor device according to at least one of Claims 1 - 6, wherein the signal processing module (11) is provided with the encapsulation (14) only on one side and has a plurality of electrically conductive contact surfaces (15.1) on a further side.

9. Sensor device according to Claim 1, wherein the protective layer (3; 13) has a slope in the direction of a limiting side surface (7.2) of the cavity (6; 16).

## Revendications

1. Dispositif capteur, comportant
- un composant de traitement du signal (1) intégré,
- un capteur sensible aux gaz (2 ; 12) relié au composant de traitement du signal (1),
- une encapsulation (4 ; 14) qui enveloppe au moins partiellement le composant de traitement du signal (1), dans lequel une cavité (6 ; 15) présente dans l'encapsulation (4 ; 14) permet d'accéder au capteur (2 ; 12) et l'encapsulation (4 ; 14) est conçue de manière à ce que la cavité (6 ; 16) s'étende du capteur (2 ; 12) jusqu'à au moins deux surfaces latérales adjacentes (7.1, 7.2) de l'encapsulation (4 ; 14), dans lequel la cavité (6 ; 16) s'étend dans l'encapsulation (4 ; 14) d'une première surface latérale (7.1) de l'encapsulation (4 ; 14) jusqu'à une surface de fond de cavité dans laquelle est disposé le capteur (2 ; 12), dans lequel les parois latérales de la cavité (6 ; 16) ne sont pas disposées perpendiculairement à la surface de base de la cavité, et
- dans lequel le capteur (2 ; 12) est réalisé sous la forme d'un capteur d'humidité et le composant de traitement du signal (1 ; 11) est conçu et réalisé de manière à convertir les signaux générés par le capteur (2 ; 12) en des valeurs de mesure d'humidité pouvant être soumises à des traitements ultérieurs, et **caractérisé en ce que** le capteur d'humidité comporte une électrode de base (2.1) sur laquelle est disposée une couche de mesure (2.3) sensible à l'humidité, sur laquelle se trouve une électrode de recouvrement (2.2) présentant une couche de protection (3 ; 13) hydrophobe et perméable à l'humidité et dans lequel le capteur d'humidité est placé sur une couche d'isolation (1.1) disposée sur le composant de traitement du signal (1 ; 11).

2. dispositif capteur selon la revendication 1, dans lequel la cavité (6 ; 16) s'étend du capteur (2 ; 12) jusqu'à deux surfaces latérales (7.1, 7.2) non parallèles de l'encapsulation (4 ; 14).

3. Dispositif capteur selon la revendication 1, dans lequel la surface de base de la cavité est réalisée sous la forme d'une parabole, dans lequel le capteur (2 ; 12) est disposé dans la zone du sommet de la parabole et les branches de la parabole s'étendent dans la direction d'une deuxième surface latérale (7.2) de l'encapsulation (4 ; 14).

4. Dispositif capteur selon la revendication 1, dans lequel
- l'encapsulation (4 ; 14) est réalisée sous forme parallélépipédique et
- la cavité (6 ; 16) s'étend dans la direction longitudinale en se rétrécissant en forme d'entonnoir d'une première surface latérale parallélépipédique (7.1) jusqu'à une surface de base de la cavité, et
- la cavité (6 ; 16) s'étend dans la direction transversale jusqu'à une deuxième surface latérale parallélépipédique (7.2), dans lequel les première et deuxième surfaces parallélépipédiques (7.1, 7.2) sont orientées de manière non parallèle l'une à l'autre.

5. Dispositif capteur selon au moins l'une des revendications précédentes, dans lequel le capteur (2 ; 12) n'est pas disposé au centre de l'encapsulation (4 ; 14).

6. Dispositif capteur selon au moins l'une des revendications précédentes, dans lequel l'encapsulation (4 ; 14) est constituée d'une résine époxyde.

7. Dispositif capteur selon au moins l'une des revendications précédentes, dans lequel le composant de traitement du signal (1) est relié à une structure de support (5) électriquement conductrice et l'encapsulation (4) entoure également la structure de support (5) à l'exception de plusieurs surfaces de contact (5.1) électriquement conductrices.

8. Dispositif capteur selon au moins l'une des revendications 1 - 6, dans lequel le composant de traitement du signal (11) est pourvu de l'encapsulation (14) sur une seule face et présente sur une autre face plusieurs surfaces de contact (15.1) électriquement conductrices.

9. Dispositif capteur selon la revendication 1, dans lequel la couche de protection (3 ; 13) présente une pente orientée dans la direction d'une surface latérale adjacente (7.2) de la cavité (6 ; 16).
